# EUROPÄISCHE PATENTSCHRIFT

(11) **EP 1 161 224 B1**
(45) Veröffentlichungstag und Bekanntmachung des Hinweises auf die Patenterteilung: **17.05.2006**
(21) Anmeldenummer: 00912570.9
(22) Anmeldetag: 09.03.2000
(51) Int. Cl.: A61K 8/02, A61K 8/03, A61K 8/72

(54) **VORRICHTUNG ZUR ABGABE KOSMETISCHER WIRKSTOFFE**
DEVICE FOR RELEASING COSMETIC ACTIVE SUBSTANCES
DISPOSITIF POUR DELIVRER DES PRINCIPES ACTIFS COSMETIQUES

(30) Priorität: 13.03.1999 DE 19911262
(43) Veröffentlichungstag der Anmeldung: 12.12.2001
(73) Patentinhaber: SCS Skin Care Systems GmbH, 56626 Andernach (DE)
(72) Erfinder: ROREGER, Michael, D-56567 Neuwied (DE)
(74) Vertreter: Schmidt, Werner
(86) Internationale Anmeldenummer: PCT/EP2000/002040
(87) Internationale Veröffentlichungsnummer: WO 2000/054744

(56) Entgegenhaltungen:
- EP-A- 0 651 984
- WO-A-98/42303
- DATABASE WPI [Online] DERWENT PUBLICATIONS LTD., LONDON, GB; access number 1994-106740, XP002141773 & JP 06 056660 A (NIPPON SURFACTANT K.K.K.) 1. März 1994 (1994-03-01)

## Beschreibung

In Medizin und Pharmazie werden Wirkstoffpflaster (Transdermale Therapeutische Systeme, TTS) zur Behandlung von insbesondere chronischen Erkrankungen des Menschen seit mehr als 20 Jahren eingesetzt. Bei solchen Pflastern, wie z. B. Herz- oder Hormonpflastern, tritt der Wirkstoff über die Haut in den menschlichen Organismus ein. Der Wirkstoff wird langanhaltend und gleichmäßig abgegeben aus Matrixschichten oder Reservoirelementen des Pflasters, welche in der Regel mit Träger- und Schutzschichten kombiniert werden.

EP-A 651 984 offenbart ein medizinisches Haftklebepflaster, dessen Rückschicht zweischichtig ist und aus einer äußeren, nicht-porösen und einer inneren, porösen Schicht besteht. In die poröse Schicht ist die Haftklebeschicht eingebettet.

In JP-A 6 056 660 wird ein Pflaster beschrieben, welches eine wässrige Lösung von Diclofenac-Natrium und Polyhydroxyalkohol-Fettsäureester, dispergiert in einem wasserhaltigen Gel enthält.

Die WO 98/42303 offenbart ein kosmetisches Pflaster, welches im trockenen Zustand nicht klebend ist und das daher vor Gebrauch angefeuchtet oder auf eine feuchte Hautstelle aufgebracht werden muss.

Auch für kosmetische Anwendungen gibt es inzwischen Wirkstoffpflaster, insbesondere für die Hautpflege, die die Wirkstoffe aber nicht durch die Haut an den Blutkreislauf abgeben, sondern nur in die Haut an epidermale und subcutane Gewebe.

Wie bei pharmazeutischen Anwendungen werden solche Pflaster dann eingesetzt, wenn zur Behandlung der Haut eine gleichmäßige Wirkstoffzufuhr über einen langen Anwendungszeitraum notwendig ist, und eine solche Wirkstoffzufuhr mit herkömmlichen, in kurzen Zeitabständen mehrmals aufzutragenden Zubereitungen wie Salben, Cremes oder Lotionen nicht zu erreichen ist.

So beschreibt US-A-5 723 138 ein klebendes Kosmetikprodukt zur Behandlung von Falten und Krähenfüßen, das in einer klebenden Schicht eine Aktivstoffmischung aus Vitamin E, Vitamin A und Aloe-vera-Extrakt enthält. EP-A-764 441 beschreibt ganz allgemein ein Patch zur gesteuerten Abgabe kosmetischer Aktivstoffe, das ein hydrophobes Reservoir umfaßt, in dem Aktivstoffpartikel dispergiert sind. Auf einem ähnlichen Produktprinzip basiert auch US-A-5 785 978, in dem eine Methode zur Behandlung von Falten beschrieben wird durch Anwendung eines Pflasters, das gepulvertes oder granuliertes Vitamin C und ggf. weitere Wirk- und Hilfsstoffe enthält.

Gerade letztgenanntes Beispiel macht die Nachteile solcher Pflaster mehr als deutlich. Dem Fachmann ist von pharmazeutischen Wirkstoffpflastern her bekannt, daß Wirkstoffe nur in gelöster oder solubilisierter Form diffusionsfähig sind und in die Haut eindringen können. Pulver und Granulate sind dazu nicht in der Lage.

Transdermale Therapiesysteme enthalten daher in der Regel Hilfsstoffe, die den abzugebenden Wirkstoff zumindest teilweise lösen, um das Eindringen von Wirkstoff in die Haut und dessen Verteilung in kutanen Geweben überhaupt erst zu ermöglichen. In der Regel werden auch noch zusätzliche Hilfsstoffe eingesetzt, die genau diesen Vorgang, die Resorption, fördern sollen.

Für kosmetische Pflaster stellt WO-A-97/48387 einen Schritt in diese Richtung dar. Es wird eine Vorrichtung in Form eines Patches für die topische Verabreichung einer Anti-Akne-Formulierung beschrieben; die Formulierung enthält mindestens zwei verschiedene Aktivstoffe und kann ggf. auch Lösungsvermittler für die Aktivstoffe umfassen. GB-A-2 265 086 beschreibt hingegen ein Patch für die perkutane Verabreichung von einem oder mehreren Aktivstoffen mit Tyrosinase-hemmender Aktivität zur Hautaufhellung. Dieses Patch kann zudem Hilfsstoffe wie Stabilisatoren, Lösungsvermittler, Hautberuhigungsmittel und Penetrationsförderer enthalten.

Aus arzneimittelrechtlichen Gründen enthalten pharmazeutische Wirkstoffpflaster (TTS) in der Regel nur einen einzigen Wirkstoff. Die abzugebende Wirkstoffmenge und damit auch die Menge an lösungsvermittelnden und resorptionsfördernden Hilfsstoffen beträgt bei den meisten auf dem Markt befindlichen Produkten nur wenige Milligramm.

In den vorgenannten Anmeldungen werden hingegen Patches beschrieben, bei denen mindestens zwei verschiedene Wirkstoffe in zum Teil relativ hohen Konzentrationen in eine klebende Schicht eingearbeitet sind. Um diese Wirkstoffe anhaltend gleichmäßig zur Erzielung einer verlängerten Wirkung in der Haut abgeben zu können, werden also mindestens zwei Lösungsvermittler und ggf. auch zwei unterschiedliche Penetrationsförderer in den erforderlichen Konzentrationen benötigt.

Genau hier liegen die Probleme und Nachteile von kosmetischen Wirkstoffpflastem nach dem Stand der Technik. Lösungsvermittler und Penetrationsförderer sind meistens flüssige Substanzen, die nicht nur den Wirkstofftransport fördern, sondern auch durchweg die unangenehme Eigenschaft haben, daß sie aufgrund ihrer physikalischen Eigenschaften klebende Schichten sehr weich machen.

Versucht man wirklich, 4 verschiedene lösungsvermittelnde und resoptionsfördemde Flüssigkeiten in ausreichender Konzentration in z. B. eine Acrylat-Klebschicht, wie in den zitierten Anmeldungen beschrieben, einzuarbeiten, dann wird diese so weich, daß sie Fäden zieht und beim Entfernen von der Haut Rückstände hinterläßt, die dann mit organischen Lösemitteln abgewaschen werden müssen.

Mit Vorrichtungen nach dem Stand der Technik können also keine gebrauchsfähigen Wirkstoffpflaster formuliert werden, bei denen die für eine kosmetische Wirksamkeit notwendige Menge an Wirkstoffen und geeigneten Hilfsstoffen direkt in eine oder mehrere Klebschichten eingearbeitet werden können. Aufgabe der vorliegenden Erfindung war es daher, Formulierungen für Klebschichten von Pflastern zu finden, die einen oder mehrere Wirkstoffe samt den geeigneten Hilfsstoffen in hoher Konzentration zumindest teilweise gelöst aufnehmen und gesteuert abgeben können, ohne dabei die Nachteile der Produkte nach dem Stand der Technik aufzuweisen.

Überraschenderweise wurde die Lösung gefunden in einem Pflaster bestehend aus Trägerschicht, Schutzschicht und druckempfindlicher Haftklebschicht zur gesteuerten Abgabe von kosmetischen Wirkstoffen an die Haut, dadurch gekennzeichnet, daß die druckempfindliche Haftklebschicht
a) 20-60 Gew.-% mindestens einer Substanz enthält, die der Schicht haftklebende Eigenschaften gibt und zur Gruppe der Polyacrylate und/oder der Ethylenvinylacetat-Copolymere gehört;
b) 4-40 Gew.-% mindestens eines Strukturpolymers enthält, das der Schicht inneren Zusammenhalt und Festigkeit gibt und zur Gruppe der Polymethacrylate, der Polyvinylpyrrolidone oder der Cellulosederivate gehört;
c) 1-20 Gew.-% mindestens einer Substanz enthält, die die zumindest teilweise Lösung von hydrophilem und/oder lipophilem kosmetischem Aktivstoff in einer haftklebenden Schicht der Zusammensetzung nach a) und b) ermöglicht und zur Gruppe der ein- und mehrwertigen Alkohole sowie deren Derivate und/oder der pflanzlichen Fette und Öle gehört;
d) 1-20 Gew.-% mindestens einer Substanz enthält, die die Aufnahme von hydrophilem und/oder lipophilem kosmetischem Aktivstoff in die Haut fördert und zur Gruppe der Fettsäuren mit einer Kettenlänge von C₈ bis C₁₈ sowie deren Ester gehört; und
e) 0,1-35 Gew.-% , vorzugsweise 0,2-30 Gew.-%, insbesondere 0,3-25 Gew.-% mindestens eines kosmetischen Aktivstoffs oder der Zubereitung mindestens eines kosmetischen Aktivstoffs umfaßt.

Druckempfindliche Haftklebeschichten im Sinne dieser Erfindung bestehen aus Haftklebern, mit Hilfe derer bei Raumtemperatur unter geringem Druck Klebschichten mit Oberflächen wie beispielsweise der menschlichen Haut zuverlässig verbunden werden können, und die einen inneren Zusammenhalt (Kohäsion) aufweisen, der ein rückstandsfreies Entfernen der Haftklebschicht und damit des Pflasters von der Anwendungsoberfläche durch einfaches Abziehen ermöglicht.

Bei kosmetischen Pflastern, bei denen kosmetische Akivstoffe aus druckempfindlichen Haftklebschichten gesteuert an die Haut abgegeben werden, kommt der Zusammensetzung der Haftklebschicht für die Prozesse der Freisetzung von kosmetischem Aktivstoff und seiner Penetration in die einzelnen Hautschichten entscheidende Bedeutung zu.

Eine der Hauptfunktionen der menschlichen Haut ist ihre Rolle als Permeabilitätsbarriere, wobei diese Eigenschaft eng mit der Zusammensetzung der Lipiddoppelschicht verbunden ist. Diese Schicht ist für lonen und die meisten polaren Substanzen nicht durchlässig. Denn zur Permeation muß eine Substanz zunächst ihre Hydrathülle verlieren, dann im Kohlenwasserstoff der Lipiddoppelschicht gelöst werden und durch diese auf die andere Seite diffundieren, wo sie wieder in Wasser gelöst oder zumindest solubilisiert wird.

Das Spektrum von Substanzen, die die zur Permeation notwendigen physikalisch-chemischen Eigenschaften aufweisen, ist außerordentlich begrenzt. Bei Substanzen, die aufgrund ihrer Eigenschaften eher ungeeignet für ein Eindringen in die Haut sind, bedient man sich daher bestimmten Hilfsstoffen bzw. komplexen Hilfssystemen (z. B. Liposomen, Nanosomen u. ä.), mittels derer die in Frage kommende Substanz durch die Barriereschichten transportiert werden sollen. Grundvoraussetzung für die Funktion eines solchen Transportsystems ist jedoch, daß der oder die Aktivstoffe zunächst aus dem Applikationsvehikel (Salbe, Creme oder auch Pflaster) freigesetzt und an der Grenzfläche Vehikel/Haut an die Haut abgegeben werden.

Dies ist aber nur dann möglich, wenn der oder die Aktivstoffe im Vehikel in einer diffusionsfähigen Form enthalten sind, das heißt gelöst oder solubilisiert sind. Das Hilfsstoffsystem für den Transport von Aktivstoff durch die Haut muß also in der Regel kombiniert werden mit einem Hilfsstoffsystem für den Transport von Aktivstoffen innerhalb des Applikationsvehikels und für die Freisetzung von Aktivstoff.

Welche der bekannten und dermatologisch ausgeprüften Hilfsstoffe für derartige Transportsysteme in Betracht kommen, hängt im Einzelfall von den Eigenschaften des jeweiligen Aktivstoffs, insbesondere der Löslichkeit, ab. Unabhängig davon können diese Hilfsstoffe selbst aber wegen ihrer physikalisch-chemischen Eigenschaften bestimmten Substanzgruppen zugeordnet werden, denen wiederum, wie eingangs erwähnt, eines gemein ist, daß sie viskositätserniedrigende und weichmachende Eigenschaften haben. Dies ist zwar einerseits Voraussetzung für die Lösungsvermittlung im Vehikel bzw. den Transport durch biologische Membranen der Haut, führt aber andererseits auch mit steigender Konzentration an solchen Hilfsstoffen zu einer zunehmenden Verflüssigung des jeweiligen Applikationsvehikels.

Das Hauptproblem bei der Formulierung einer funktionsfähigen Haftklebschicht für Pflaster zur Abgabe von kosmetischen Aktivstoffen bestand also darin, Lösungs- bzw. Lösungsvermittlungs- und Transportsysteme für Aktivstoffe mit Kleberhilfsstoffen so zu kombinieren, daß Eigenschaften und Funktionsfähigkeit der Haftklebschicht durch Viskositätsemiedrigung nicht nachhaltig beeinträchtigt werden.

Eine generelle Lösung für dieses Problem konnte nicht gefunden werden. Eine stabile und funktionsfähige Haftklebschicht wird nur dann erhalten, wenn bestimmte Substanzklassen von Lösungsmitteln oder Lösungsvermittlern in Mischung mit einer bestimmten Substanzklasse von Transportmitteln mit ausgewählten Haftklebern kombiniert und durch zusätzliche Strukturbildner für den inneren Zusammenhalt verfestigt wird. So kann das gewünschte Ergebnis beispielsweise mit den weitverbreiteten Haftklebstoffen aus der Gruppe der Natur- und Synthesekautschuke und deren Derivate nicht erreicht werden.

Die druckempfindliche Haftklebeschicht enthält daher 20-60 Gew.-%, vorzugsweise 25-55 Gew.-%, insbesondere 30-50 Gew.-% mindestens einer Substanz, die der Schicht haftklebende Eigenschaften gibt und zur Gruppe der Polyacrylate und/oder der Ethylen-Vinylacetat-Copolymere gehört.

Polyacrylate, Polymethacrylate und deren Copolymere gehören vorzugsweise zur Gruppe der Alkylesterpolymere, wie beispielsweise Butylacrylat, Isobutylacrylat, Hexylacrylat, Octylacrylat, 2-Ethylhexylacrylat, Isooctylacrylat, Methylmethacrylat, Ethylmethacrylat, Butylmethacrylat, Isobutylmethacrylat, 2-Ethylhexylmethacrylat oder Isooctylmethacrylat.

Desweiteren enthält die druckempfindliche Haftklebeschicht 4-40 Gew.-%, vorzugsweise 6-35 Gew.-%, insbesondere 8-30 Gew.-% mindestens eines Strukturpolymers, das der Schicht inneren Zusammenhalt und Festigkeit gibt und zur Gruppe der Polymethacrylate, der Polyvinylpyrrolidone oder der Cellulosederivate gehört. Als Strukturpolymer aus der Gruppe der Polymethacrylate ist besonders Poly(butylmethacrylat-methylmethacrylat) bevorzugt.

Geeignete Cellulosederivate sind bespielsweise Methylcellulose, Ethylcellulose, Hydroxypropylcellulose, Hydroxyethylcellulose, Hydroxypropylmethylcellulose, Natriumcarboxymethylcellulose, Celluloseacetatphthalat, Hydroxypropylmethylcellulosephthalat, Ethylcarboxyethylcellulose, Celluloseacetatsuccinat oder Ethylcellulosesuccinat.

Die druckempfindliche Haftklebschicht enthält zudem 1-20 Gew.-%, vorzugsweise 2-18 Gew.-%, insbesondere 3-15 Gew.-% mindestens einer Substanz, die die zumindest teilweise Lösung von hydrophilem und/oder lipophilem kosmetischem Aktivstoff in der druckempfindlichen Haftklebschicht ermöglicht und zur Gruppe der ein- und mehrwertigen Alkohole sowie deren Derivate und/oder der pflanzlichen Fette und Öle gehört.

Geeignete ein- und mehrwertige Alkohole und deren Derivate sind beispielsweise Ethanol, Propanol, Isopropanol, Butanol, Octanol, Decanol, Dodecanol, Cetylalkohol, Propylenglycol, 1,3-Butylenglykol, Glycerin, Polyethylenglykol, Polypropylenglykol oder Polyethylenglykol-Polypropylenglykol-Blockpolymerisate. Zur Solubilisierung von kosmetischem Aktivstoff geeignete pflanzliche Fette und Öle können beispielsweise Mandelöl, Erdnussöl, Sojaöl, Leinöl, Jojobaöl, Avocadoöl, Olivenöl, Rizinusöl, Palmkemöl, Rapsöl, Sesamöl, Aprikosenöl, Pfirsichkernöl, Weizenkeimöl, Maiskeimöl, Mohnöl, Saffloröl, Sonnenblumenöl oder Baumwollsaatöl sein.

Desweiteren enthält die druckempfindliche Haftklebeschicht 1-20 Gew.-%, vorzugsweise 2-18 Gew.%, insbesondere 3-15 Gew.-% mindestens einer Substanz, die die Aufnahme von hydrophilem und/oder lipophilem kosmetischem Aktivstoff in die Haut fördert (Resorptionsförderer, Enhancer) und zur Gruppe der Fettsäuren mit einer Kettenlänge von C₈ - C₁₈ wie deren Ester gehört.

Geeignete Fettsäuren können gesättigte Fettsäuren sein, wie beispielsweise Caprylsäure, Caprinsäure, Laurinsäure, Myristinsäure, Palmitinsäure, Stearinsäure oder ungesättigte Fettsäuren wie Ölsäure, Linolsäure oder Linolensäure. Aus der großen Gruppe der C₈ - C₁₈ Fettsäureester sind vorzugsweise geeignet Isopropylmyristat, Ethyloleat, Methyllaurat, Polyethylenglykolmonolaurat, Glycerolmonolaurat, Glycerolmonocaprylat und Propylenglycoldicaprylat. Die letztgenannten Caprylsäureester eignen sich insbesondere zur Resorptionsförderung wasserlöslicher Aktivstoffe.

Die Trägerschicht des Pflasters kann permeabel oder okklusiv sein und beispielsweise aus einer Kunststoffolie, einem Papier, einem Gewebe oder einem Vlies bestehen.

Geeignete Materialien für die flexible Rückschicht sind beispielsweise Polyester, Polyamid, Polyethylen, Polypropylen, Polyurethan, Polyvinylchlorid, und zwar sowohl als sogenannte Solofolien als auch als Sandwichfolien in Kombination von Folien aus verschiedenen dieser Kunststoffe. Die Folien können außerdem mit Aluminium bedampft bzw. mit einer Aluminiumfolie kaschiert sein.

Geeignete Materialien für die ablösbare Schutzschicht sind bespielsweise Polyester, Polyethylen, Polypropylen sowie Papiere, die mit diesen Materialien beschichtet und ggf. mit Aluminium bedampft bzw. mit einer Aluminiumfolie kaschiert werden. Außerdem sind die Folien bzw. Papiere mit Silikon beschichtet, um ihnen die wiederablösbaren Eigenschaften zu verleihen.

Die in der druckempfindlichen Haftklebschicht enthaltenen kosmetischen Aktivstoffe dienen vorzugsweise
- der Hautbefeuchtung wie beispielsweise Milchsäure, Glykolsäure, Asparaginsäure, Pyrrolidoncarbonsäure, Harnstoff, Glycin, Serin und Extrakte aus Aloe-vera oder Algen
- der Faltenbehandlung wie beispielsweise Hyaluronsäure, Chitosan, Kollagen oder Ascorbinsäure
- der Behandlung von Alterserscheinungen der Haut wie beispielsweise Vitamin A und E
- der Behandlung von Pickeln wie beispielsweise Salicylsäure, Schwefel oder Benzoylperoxid
- der Hautaufhellung wie beispielsweise Hydrochinon oder Kojisäure
- der Hautstraffung wie Extrakte aus Gingko biloba, Hafer, Centella asiatica oder Echinacea purpurea
- der Regeneration und Revitalisierung der Haut wie beispielsweise Vitamine, Ceramide, Glykoproteine oder Squalene
- der Hautberuhigung und Entzündungshemmung Extrakte aus Hypericum perforatum oder Matricaria chamomilla.

Die Aktivstoffe können allein oder in Mischung in der Haftklebschicht eingearbeitet werden, können aber auch in Form gebrauchsfertiger Zubereitungen, vorzugsweise Lösungen, Extrakten, Lotionen, Salben, Cremes oder Gelen eingearbeitet werden.

Die in der druckempfindlichen Haftklebschicht enthaltenen kosmetischen Aktivstoffe dienen vorzugsweise der Hautbefeuchtung, der Faltenbehandlung, der Behandlung von Alterserscheinungen der Haut, der Behandlung von Pickeln, der Hautaufhellung, der Hautstraffung oder der Regeneration und Revitalisierung der Haut.

Zur gezielten Einstellung bestimmter Freisetzungseigenschaften oder auch besonderer Eigenschaften des Pflasters selbst, wie beispielsweise Stärke der Klebkraft oder Dauer der Haftung auf der Haut, kann die druckempfindliche Haftklebschicht weitere Hilfsstoffe wie viskositätserhöhende Substanzen, Irritationsminderer und Hautberuhigungsmittel, Feuchthaltemittel, Weichmacher, Konservierungsmittel, Desinfektionsmittel, pH-Regulatoren, Antioxidantien, Duftstoffe, Farbstoffe, Füllstoffe oder Klebharze umfassen.

Zu den viskositätserhöhenden Substanzen gehören vorzugsweise Gelatine, pflanzliche Polysaccharide wie Alginate, Pektine, Carrageenane oder Xanthan, Cellulosederivate wie Methylcellulose, Hydroxypropycellulose, Hydroxyethylcellulose, Hydroxypropylmethylcellulose oder Natriumcarboximethylcellulose, Stärke und Stärkederivate, Galaktomannan und Galaktomannanderivate, Polyvinylalkohol, Vinylpyrrolidon-Vinylacetat-Mischpolymerisate, Polyethylenglykole und Polypropylenglykole.

Irritationsminderer und Hautberuhigungsmittel sind beispielsweise Rutin, Squalen, Kamillenextrakt, Bisabolol, Chamazulen, Guajazulen, Farnesol, Sitosterin, Sitosterolglykosid, Glycyrrhetinsäure und deren Ester.

Als Feuchthaltemittel kann die Haftklebschicht beispielsweise Glycerin, Propylenglykol, Sorbitol, Mannitol, Polyethylenglykol oder Polypropylenglykol umfassen.

Als Weichmacher kann die druckempfindliche Haftklebschicht Citronensäureester wie Triethylcitrat oder Acetyltriethylcitrat, Weinsäureester wie Dibutyltartrat, Glycerinester wie Glycerindiacetat oder Glycerintriacetat, Phthalsäureester wie Dibutylphthalat oder Diethylphthalat und/oder hydrophile Tenside, vorzugsweise hydrophile, nichtionogene Tenside, wie bspw. Partialfettsäureester von Zuckern, Polyethylenglykolfettsäureester, Polyethylenglykolfettalkoholether oder Polyethylenglykolsorbitanfettsäureester enthalten.

Als Konservierungsmittel können bspw. p-Cl-m-Kresol, Phenylethylalkohol, Phenoxyethylalkohol, Chlorbutanol, 4-Hydroxybenzoesäuremethylester, 4-Hydroxybenzoesäurepropylester, Benzalkoniumchlorid, Cetylpyridiniumchlorid, Chlorhexidindiacetat oder -gluconat, Ethanol oder Propylenglykol verwendet werden.

Als Desinfektionsmittel können z. B. Halogene wie Polyvidon-Jod, Halogenverbindungen wie Natriumhypochlorid oder Tosylchlorid-Natrium, Oxidationsmittel wie Wasserstoffperoxid oder Kaliumpermanganat, Arylquecksilberverbindungen wie Phenylmercuriborat oder Merbromin, Alkylquecksilberverbindungen wie Thiomersal, Organozinnverbindungen wie Tri-n-butylzinnbenzoat, Silbereiweißverbindungen wie Silbereiweiß-acetyltannat, Alkohole wie Ethanol, n-Propanol oder Isopropanol, Phenole wie Thymol, o-Phenylphenol, 2-Benzyl-4-chlorphenol, Hexachlorophen oder Hexylresorcin oder organische Stickstoffverbindungen wie 8-Hydroxychinolin, Chlorquinaldol, Clioquinol, Ethacridin, Hexetidin, Chlorhexidin oder Ambazon verwendet werden.

Glycerinpuffer, Citratpuffer, Boratpuffer oder Citronensäure-Phosphat-Puffer beispielsweise können als pH-Regulatoren verwendet werden.

Als Antioxidantien können beispielsweise Ascorbinsäure, Ascorbylpalmitat, Tocopherolacetat, Propylgallat, Butylhydroxyanisol oder Butylhydroxytoluol verwendet werden.

Als Füllstoffe können z. B. mikrokristalline Cellulose, Aluminiumoxid, Zinkoxid, Titanoxid, Talkum, Siliciumdioxid, Magnesiumsilikat, Magnesium-Aluminiumsilikat, Kaolin, hydrophobe Stärke, Calciumstearat oder Calciumphosphat verwendet werden.

Als Harze können z. B. Epoxidharze, Melaminharze, Phenol-Formaldehyd-Harze und Resorcin-Formaldehyd-Harze verwendet werden, wobei auch unter anderem die nachfolgend genannten modifizierten Harze eingesetzt werden können: Hydriertes Kolophonium, polymerisiertes Kolophonium, dimerisierte Harzsäuren, disproportioniertes Kolophonium, Methylester von Kolophonium, Glycerinester von hydriertem Kolophonium, Methylester von hydriertem Kolophonium, Pentalester, Triethylenglycolester von hydriertem Kolophonium, Hydroabietylalkohol und dessen Derivate, Glycerinester, Di-Triolester und Pentalester von Harzsäuren, Pentalester von polymerisiertem Kolophonium, Pentalester von dimerisiertem Kolophonium, Glycerinester von dimerisiertem Kolophonium, Ester von Maleinsäure- oder Phenolmodifiziertem Kolophonium, aromatische und aliphatische Kohlenwasserstoffharze, hydrierte Harze, Polyterpenharze und modifizierte Terpenharze.

Die druckempfindliche Haftklebschicht des erfindungsgemäßen Pflasters wird vorzugsweise einschichtig formuliert, kann aber auch mehrschichtig aufgebaut sein, wobei zumindest in einer Schicht kosmetischer Aktivstoff enthalten ist.

Ein mehrschichtiger Aufbau kann beispielsweise erforderlich sein, wenn zur Erzielung eines bestimmten Freisetzungsprofils mit anhaltender Freisetzung von kosmetischem Aktivstoff über einen verlängerten Zeitraum Schichten mit unterschiedlichen Aktivstoffkonzentrationen nach bekannten Verfahren zu einem Verbund zusammengefügt werden. Aus dem gleichen Grund kann eine aktivstoffhaltige Haftklebschicht mit einer aktivstofffreien kombiniert werden, die dann als Steuerelement für die Freisetzung fungiert. Auch im Fall, daß unterschiedliche Aktivstoffe unterschiedlich freigesetzt werden sollen, bietet sich ein mehrschichtiger Aufbau der Haftklebschicht des erfindungsgemäßen Pflasters an.

Zur Herstellung der druckempfindlichen Haftklebschicht kann die dazu notwendige Klebmasse als organische Lösung, als wässrige Dispersion oder, wie auch die Beispiele zeigen, bei temperaturunempfindlichen Aktivstoffen als löse- und dispersionmittelfreie Schmelze formuliert werden. Die Klebmassen können nach dem Fachmann bekannten Verfahren durch Extrusion, Gießen, Aufsprühen, Aufdrucken, Walzenauftrag oder Rakelauftrag aus Lösung, Dispersion oder Schmelze auf ein geeignetes Substrat, vorzugsweise die Schutzschicht des erfindungsgemäßen Pflasters verarbeitet werden. Nach dem Trocknen oder Abkühlen wird die resultierende druckempfindliche Haftklebschicht vorzugsweise direkt mit der Trägerschicht des Pflasters kombiniert.

Mögliche Formulierungen und Verfahren zur Herstellung des erfindungsgemäßen Pflasters zur gesteuerten Abgabe von kosmetischem Aktivstoff an die Haut entsprechend den Merkmalen des Hauptanspruchs werden im folgenden beispielhaft erläutert, ohne daß die Erfindung darauf beschränkt wäre.

### Beispiel 1

In 44,5 g Ethylacetat werden 31,5 g des Strukturpolymers Poly(butylmethacrylatmethylmethacrylat) gelöst. Die Lösung wird in 111 g einer 50%igen Lösung des Haftklebpolymers Polyethylhexylacrylat in Ethylacetat eingerührt. In die Lösung von Strukturpolymer und Haftklebpolymer werden nacheinander 1 g des Strukturpolymers Polyvinylpyrrolidon, 2 g Propylenglykoldicaprylat (Resorptionsförderer für hydrophilen kosmetischen Aktivstoff), 2 g Glycerin und 3 g Propylenglykol sowie 2,5 g Aloe vera-Extrakt und 2,5 g Algenextrakt eingerührt.

Die homogene Masse wird mit einem Flächengewicht von 200 g/m² mittels Walzenauftrag auf eine silikonisierte Polyesterfolie beschichtet und bei einer Temperatur von 60 °C in einem Umlufttrockenkanal getrocknet. Nach dem Trocknen wird eine Polyethylen-Trägerfolie auf die getrocknete, druckempfindliche Haftklebschicht kaschiert.

Aus dem Laminat werden bananenförmige Pflaster gestanzt. Über Nacht unter die Augen geklebt, setzen die Pflaster kontinuierlich über mindestens 10 Stunden die hydrophilen Aktivstoffe zur Verringerung der Tiefe von Hautfalten frei.

### Beispiel 2

In 47 g Ethylacetat werden 31 g des Strukturpolymers Poly(butylmethacrylatmethylmethacrylat) gelöst. Die Lösung wird in 106 g einer 50%igen Lösung des Haftklebpolymers Polyethylhexylacrylat in Ethylacetat eingerührt. In die Lösung von Strukturpolymer und Haftklebpolymer werden nacheinander 3 g des Strukturpolymers Ethylcellulose, 5 g Sojaöl, 2 g Ölsäure, 2 g Isopropylmyristat, 2 g Retinylpalmistat und 2 g α-Tocopherolacetat eingerührt.

Die homogene Masse wird mit einem Flächengewicht von 200 g/m² mittels Walzenauftrag auf eine silikonisierte Polyesterfolie beschichtet und bei einer Temperatur von 60 °C in einem Umlufttrockenkanal getrocknet. Nach dem Trocknen wird eine Polyurethan-Trägerfolie auf die getrocknete, druckempfindliche Haftklebschicht kaschiert.

Aus dem Laminat werden Pflaster in Formen für spezifische Gesichtspartien, z. B. den Stirnbereich oder den Kinnbereich, ausgestanzt. Über Nacht angewandt, setzen die Pflaster kontinuierlich über mindestens 10 Stunden die lipophilen Aktivstoffe zur Behandlung von Alterserscheinungen der Haut frei.

### Beispiel 3

Zusammen mit 15 g eines Methylesters von teilhydriertem Kolophonium und 35 g hydriertem Kolophonium werden 25 g eines Ethylen-Vinylacetat-Copolymers mit einem Vinylacetatgehalt von 28 % und 10 g eines Ethylen-Vinylacetat-Copolymers mit einem Vinylacetatgehalt von 40 % bei 120 °C klar geschmolzen und dann auf 90 °C abgekühlt.
In die Schmelze werden nacheinander 2 g Ethylcellulose, 2,5 g Propylenglykol, 2 g Glycerin, 1 g polyethoxyliertes hydriertes Rizinusöl, 2 g Polyethylenglykolmonolaurat, 2 g Glycerylmonocaprylat, 1 g Milchsäure, 0,5 Harnstoff, 1 g Ginkgo-Extrakt und 1 g Hafer-Extrakt langsam eingerührt, bis eine gleichmäßige Verteilung erreicht ist.
Die homogene Masse wird mit einem Flächengewicht von 250 g/m² mittels Rakelauftrag auf eine silikonisierte Polyesterfolie beschichtet. Nach dem Erhalten der druckempfindlichen Haftklebschicht wird eine Polyetlylen-Trägerfolie auflaminiert, und aus dem Laminat werden Pflaster der gewünschten Fläche ausgestanzt.

Nach dem Aufkleben auf die Haut werden aus der Haftklebschicht kontinuierlich die Aktivstoffe mit hautbefeuchtender und hautstraffender Wirkung freigesetzt.

### Beispiel 4

Zusammen mit 50 g Hydroabietylalkohol und 8 g eines Pentaerythritylesters von teilhydriertem Kolophonium werden 23 g eines Ethylenvinylacetat-Copolymers mit einem Vinylacetatgehalt von 28 % bei 120 °C klar geschmolzen und dann auf 90 °C abgekühlt. In der Schmelze werden nacheinander 3 g Ethylcellulose, 3 g Sesamöl, 2 g Dodecanol, 2 g Kamillenextrakt, 1 g Johanniskrautextrakt und 0,2 g Rutin langsam eingerührt, bis eine gleichmäßige Verteilung erreicht ist.

Die homogene Masse wird mit einem Flächengewicht von 250g/m² mittels Rakelauftrag auf eine silikonisierte Polyesterfolie beschichtet. Nach dem Erkalten der druckempfindlichen Haftklebschicht wird eine Polyethylen-Trägerfolie auflaminiert, und aus dem Laminat werden Pflaster in gewünschten Fläche ausgestanzt.

Die Pflaster werden auf strapazierte Hautpartien geklebt und setzen aus der Haftklebschicht kontinuierlich die hautberuhigenden, entzündungshemmenden und revitalisierenden Aktivstoffe frei.

### Beispiel 5

In 51,89 g Ethylacetat werden 29 g des Strukturpolymers Poly(butylmethacrylatmethylmethacrylat) gelöst. Die Lösung wird in 111,7 g einer 50 %igen Lösung des Haftklebepolymers Polyhexylacrylat in Ethylacetat eingerührt. In die Lösung von Strukturpolymer und Haftklebepolymer werden nacheinander 1 g Salicylsäure, 6 g Glycerol (Resorptionsförderer für hydrophile kosmetische Aktivstoffe) 1,5 g Salbei-Extrakt, 1,3 g Hamamelis-Extrakt, 1,2 g Beinwell-Extrakt, 1,1 g Efeu-Extrakt, 1 g Kamille-Extrakt und 0,9 g Aloe-Extrakt eingerührt.

Die homogene Masse wird mit einem Flächengewicht von 200 g/m² mittels Walzenauftrag auf eine silikonisierte Polyesterfolie beschichtet und bei einer Temperatur von 60 °C in einem Umlufttrockenkanal getrocknet. Nach dem Trocknen wird eine Polyurethan-Trägerfolie auf die getrocknete, druckempfindliche Haftklebschicht kaschiert. Aus dem Laminat werden kleine, runde Pflaster ausgestanzt.

Über Nacht auf Pickel oder Mitesser geklebt, setzen die Pflaster kontinuierlich über mindestens 10 Stunden die reinigenden, antibakteriellen und hautberuhigenden Aktivstoffe frei.

### Beispiel 6

In 54,05 g Ethylacetat werden 30 g des Strukturpolymers Poly(butylmethacrylatmethylmethacrylat) gelöst. Die Lösung wird in 110,78 g einer 50 %igen Lösung des Haftklebepolymers Polyhexylacrylat in Ethylacetat eingerührt. In die Lösung von Strukturpolymer und Haftklebepolymer werden nacheinander 5 g Wasser, 5 g Glycerol (Resorptionsförderer für hydrophile kosmetische Aktivstoffe) 1 g Magnesium-Ascorbyl-Phosphat, 0,5 g Kojisäure, und 0,2 g Arbutin eingerührt.

Die homogene Masse wird mit einem Flächengewicht von 200 g/m² mittels Walzenauftrag auf eine silikonisierte Polyesterfolie beschichtet und bei einer Temperatur von 60 °C in einem Umlufttrockenkanal getrocknet. Nach dem Trocknen wird eine Polyurethan-Trägerfolie auf die getrocknete, druckempfindliche Haftklebeschicht kaschiert. Aus dem Laminat werden kleine, runde Pflaster ausgestanzt.

Über Nacht auf Pigmentflächen oder Hautverfärbungen geklebt, setzen die Pflaster kontinuierlich über mindestens 10 Stunden die hautbleichenden Aktivstoffe frei.

## Patentansprüche

1. Pflaster, bestehend aus Trägerschicht, Schutzschicht und druckempfindlicher Haftklebschicht zur gesteuerten Abgabe von kosmetischen Wirkstoffen an die Haut, **dadurch gekennzeichnet, daß** die druckempfindliche Haftklebeschicht
a) 20-60 Gew.-% mindestens einer Substanz enthält, die der Schicht haftklebende Eigenschaften gibt und zur Gruppe der Polyacrylate und/oder der Ethylen-Vinylacetat-Copolymere gehört;
b) 4-40 Gew.-% mindestens eines Strukturpolymers enthält, das der Schicht inneren Zusammenhalt und Festigkeit gibt und zur Gruppe der Polymethacrylate, der Polyvinylpyrrolidone oder der Cellulosederivate gehört;
c) 1-20 Gew.-% mindestens einer Substanz enthält, die die zumindest teilweise Lösung von hydrophilem und/oder lipophilem kosmetischem Aktivstoff in einer haftklebenden Schicht der Zusammensetzung nach a) und b) ermöglicht und zur Gruppe der ein- und mehrwertigen Alkohole sowie deren Derivate und/oder der pflanzlichen Fette und Öle gehört;
d) 1-20 Gew.-% mindestens einer Substanz enthält, die die Aufnahme von hydrophilem und/oder lipophilem kosmetischem Aktivstoff in die Haut fördert und zur Gruppe der Fettsäuren mit einer Kettenlänge von C₈ bis C₁₈ sowie deren Ester gehört; und
e) 0, 1-35 Gew.-% mindestens eines kosmetischen Aktivstoffs oder der Zubereitung mindestens eines kosmetischen Aktivstoffs umfaßt.

2. Pflaster gemäß Anspruch 1, **dadurch gekennzeichnet, daß** die in der druckempfindlichen Haftklebschicht enthaltenen kosmetischen Aktivstoffe vorzugsweise der Hautbefeuchtung, der Faltenbehandlung, der Behandlung von Alterserscheinungen der Haut, der Behandlung von Pickeln, der Hautaufhellung, der Hautstraffung oder der Regeneration und Revitalisierung der Haut dienen.

3. Pflaster gemäß Anspruch 1 oder 2, **dadurch gekennzeichnet, daß** die druckempfindliche Haftklebschicht die Zubereitung eines kosmetischen Aktivstoffs enthält, die vorzugsweise eine Lösung, ein Extrakt, eine Lotion, eine Salbe, eine Creme oder ein Gel ist.

4. Pflaster gemäß einem oder mehreren der Ansprüche 1 bis 3, **dadurch gekennzeichnet, daß** die druckempfindliche Haftklebschicht weitere Hilfsstoffe wie viskositätserhöhende Substanzen, Irritationsminderer und Hautberuhigungsmittel, Feuchthaltemittel, Weichmacher, Konservierungsmittel, Desinfektionsmittel, pHRegulatoren, Antioxidantien, Duftstoffe, Farbstoffe, Füllstoffe oder Klebharze umfaßt.

5. Pflaster gemäß einem oder mehreren der Ansprüche 1 bis 4, **dadurch gekennzeichnet, daß** die druckempfindliche Haftklebschicht ein Laminat aus mehreren Schichten ist, wobei zumindest in einer Schicht kosmetischer Aktivstoff enthalten ist.

6. Pflaster gemäß einem oder mehreren der Ansprüche 1 bis 5, **dadurch gekennzeichnet, daß** die druckempfindliche Haftklebschicht aus Lösung, Dispersion oder Schmelze durch Extrusion, Gießen, Aufsprühen, Aufdrucken, Rakelauftrag oder Walzenauftrag hergestellt wird.

7. Pflaster gemäß einem oder mehreren der Ansprüche 1 bis 6, **dadurch gekennzeichnet, daß** das Strukturpolymer Poly(butylmethacrylatmethylmethacrylat) ist.

## Claims

1. A patch comprising backing layer, protective layer and pressure-sensitive adhesive layer for the controlled delivery of cosmetic active substances to the skin, wherein said pressure-sensitive adhesive layer comprises
a) 20-60% by weight of at least one substance which gives the layer adhesive properties and belongs to the group of polyacrylates and/or ethylene-vinyl acetate copolymers;
b) 4-40% by weight of at least one structural polymer which gives the layer internal cohesion and strength and belongs to the group of polymethacrylates, acrylate/methacrylate polyvinylpyrrolidones, or cellulose derivatives;
c) 1-20% by weight of at least one substance which permits the at least partial dissolution of hydrophilic and/or lipophilic cosmetic active substance in an adhesive layer of the composition according to a and b and which belongs to the group of monohydric and polyhydric alcohols and derivatives thereof and/or vegetable fats and oils;
d) 1-20% by weight of at least one substance which promotes the absorption of hydrophilic and/or lipophilic cosmetic active substance into the skin and belongs to the group of fatty acids having a chain length from C₈ to C₁₈ and esters thereof; and
e) 0.1-35% by weight of at least one cosmetic active substance or the preparation of at least one cosmetic active substance.

2. The patch as claimed in claim 1, wherein the cosmetic active substances present in said pressure-sensitive adhesive layer serve primarily for skin moisturizing, for treating wrinkles, for treating skin aging phenomena, for treating spots, for skin lightening, for skin firming or for regenerating and revitalizing the skin.

3. The patch as claimed in claim 1 or 2, wherein said pressure-sensitive adhesive layer comprises the cosmetic active substance preparation which is preferably a solution, extract, lotion, ointment, cream, or gel.

4. The patch as claimed in one or more of claims 1 to 3, wherein said pressure-sensitive adhesive layer comprises further auxiliaries such as viscosity-increasing substances, antiirritants and skin relief agents, humectants, plasticizers, preservatives, disinfectants, pH regulators, antioxidants, fragrances, colorants, fillers, or tackifier resins.

5. The patch as claimed in one or more of claims 1 to 4, wherein said pressure-sensitive adhesive layer is a laminate of two or more layers at least one of which contains cosmetic active substance.

6. The patch as claimed in one or more of claims 1 to 5, wherein said pressure-sensitive adhesive layer is prepared from a solution, dispersion or melt by extrusion, pouring, spraying, printing, blade application or roller application.

7. The patch as claimed in one or more of claims 1 to 6, wherein said structural polymer is poly(butyl methacrylate-methyl methacrylate).

## Revendications

1. Pansement constitué par une couche substrat, une couche protectrice et une couche adhésive sensible à la pression pour la libération contrôlée de principes actifs cosmétiques sur la peau, **caractérisé en ce que** la couche adhésive sensible à la pression comprend
a) de 20 à 60 % en masse d'au moins une substance qui confère à la couche des propriétés adhésives et appartient au groupe des polyacrylates et/ou des copolymères éthylène-acétate de vinyle ;
b) de 4 à 40 % en masse d'au moins un polymère de structure qui consolide l'intérieur de la couche et lui confère une solidité et appartient au groupe des polyméthacrylates, des polyvinylpyrrolidones ou des dérivés de la cellulose ;
c) de 1 à 20 % en masse d'au moins une substance qui permet la dissolution au moins en partie du principe actif cosmétique hydrophile et/ou lipophile dans une couche adhésive de la composition selon a) et b) et appartient au groupe des alcools mono- et polyfonctionnels, ainsi que de leurs dérivés et/ou des graisses et huiles végétales ;
d) de 1 à 20 % en masse d'au moins une substance qui favorise l'absorption du principe actif cosmétique hydrophile et/ou lipophile dans la peau et appartient au groupe des acides gras présentant une chaîne d'une longueur de C₈ à C₁₈ ainsi que de leurs esters ; et
e) de 0,1 à 35 % en masse d'au moins un principe actif cosmétique ou de la préparation d'au moins un principe actif cosmétique.

2. Pansement selon la revendication 1, **caractérisé en ce que** les principes actifs contenus dans la couche adhésive sensible à la pression servent de préférence à l'humidification de la peau, au traitement des rides, au traitement des phénomènes de vieillissement de la peau, au traitement de boutons, à la clarification de la peau, au raffermissement de la peau ou à la régénération et à la revitalisation de la peau.

3. Pansement selon la revendication 1 ou 2, **caractérisé en ce que** la couche adhésive sensible à la pression renferme la préparation d'un principe actif cosmétique, qui est de préférence une solution, un extrait, une lotion, une pommade, une crème ou un gel.

4. Pansement selon l'une des revendications 1 à 3, **caractérisé en ce que** la couche adhésive sensible à la pression renferme d'autres adjuvants tels que les substances augmentant la viscosité, les substances atténuant l'irritation et les agents calmants pour la peau, les agents humidifiants, les assouplissants, les agents de conservation, les agents désinfectants, les régulateurs du pH, les antioxydants, les parfums, les colorants, les charges ou les résines adhésives.

5. Pansement selon l'une des revendications 1 à 4, **caractérisé en ce que** la couche adhérente sensible à la pression est un stratifié de plusieurs couches, un principe actif cosmétique étant contenu dans au moins une couche.

6. Pansement selon l'une des revendications 1 à 5, **caractérisé en ce que** la couche adhérente sensible à la pression est préparée à partir d'une solution, d'une dispersion ou d'une matière fondue par extrusion, par coulée, par pulvérisation, par apposition, par application à la racle ou par laminage.

7. Pansement selon l'une des revendications 1 à 6, **caractérisé en ce que** le polymère de structure est le poly(méthacrylate de butyle-méthacrylate de méthyle).
